# EUROPEAN PATENT APPLICATION

(11) **EP 3 056 206 A1**
(43) Date of publication of application: **17.08.2016**
(21) Application number: 14851908.5
(22) Date of filing: 11.10.2014
(51) Int. Cl.: A61K 31/519, A61K 9/06, A61P 35/00, A61P 17/00

(54) **ICOTINIB-CONTAINING TOPICAL SKIN PHARMACEUTICAL COMPOSITION AND USE THEREOF**

(30) Priority: 11.10.2013 WO PCT/CN2013/085042
(71) Applicant: Betta Pharmaceuticals Co., Ltd., Yuhang Hangzhou, Zhejiang 311100 (CN)
(72) Inventor: WANG, Yinxiang, Beijing 100176 (CN); YUAN, Shujun, Beijing 100176 (CN); WANG, Yanping, Beijing 100176 (CN); HU, Shaojing, Beijing 100176 (CN); HU, Yunyan, Beijing 100176 (CN)
(74) Representative: Walsh, Marie Goretti
(86) International application number: PCT/CN2014/088344
(87) International publication number: WO 2015/051763

(57) **Abstract**

Disclosed are topical preparations for inhibiting tyrosine kinase and preparation methods thereof, and especially topical pharmaceutical compositions for inhibiting tyrosine kinase and preparation methods thereof. The active ingredient of the topical preparations is Icotinib or a pharmaceutically acceptable salt thereof. The preparations are suitable for topical application, with minimal skin irritation, no adverse reactions such as pruritus, burning sensations, tingling, dry skin, erythema and rashes, without parahormone-related side effects such as skin atrophy, pigmentation or hypopigmentation for long-term use, as well as no related dermatological symptoms after drug withdrawal. The preparation methods are easily understood, operable, and controllable, and are suitable for industry mass production.

## Description

### FIELD OF THE INVENTION

The present invention relates to topical skin pharmaceutical compositions containing Icotinib or its pharmaceutically acceptable salts and uses thereof.

### BACKGROUND OF THE INVENTION

Dermatologic diseases relate to skin disorders. Some of the common and frequently-occurring diseases seriously affecting people's health are leprosy, scabies, fungal disease, psoriasis, and skin bacterial infections. In dermatologic diseases, the morphologies, structures and functions of skin (including hairs and nails) change after they are affected by internal and external factors, resulting in the pathological process, and accordingly generating a variety of clinical manifestations. The incidence of dermatologic diseases is very high. While the majority of them are mild and often are not life-threatening (only a few are severer and life threatening), they can seriously affect the appearance of patients, causing a severe psychological burden, thereby affecting the patients' daily works and lives.

Among them, psoriasis is a common chronic inflammatory skin disease, the incidence rate of which is about 2% in Western countries and about 0.3% in Asia. The incidence rate, however, has increased rapidly in recent years. The typical skin manifestations of psoriasis include demarcated red patches with silvery white scales, which can seriously affect patients' appearance. Thus, although psoriasis is not life-threatening, it causes a heavy psychological burden, thereby affecting the patient's daily works and lives. Improper treatment can make it worse and increase the psychological and economic burdens of the patients. For example, erythrodermic and pustular psoriasis can cause metabolic disorders of the whole body, multiple organ (such as cardiovascular and lung) complications, and infections, which can be life-threatening. Although some of them are caused by unknown etiology, a considerable amount of erythrodermic and pustular psoriasis, including onset and exacerbation, is caused by improper treatment.

Early studies suggest that the pathogenesis of psoriasis is mainly caused by the abnormal differentiation of epidermal hyperplasia and the activation of the immune system. Tazarotene (a vitamin A derivative), lente and potent glucocorticoid, and calcipotriol (a Vitamin D3 derivative) are recommended as topical treatments according to China's psoriasis treatment guidelines of 2008. However, the long-term use of corticosteroids can cause many side effects, including skin atrophy, telangiectasia, folliculitis, pigmentation and hypopigmentation. Long-term use of potent glucocorticoid preparations may cause systemic reactions, and even induced pustular or erythrodermic psoriasis after withdrawal. The vitamin A derivatives can irritate a mucocutaneous zone, especially for patients prone to allergies. Common side effects of calcipotriol include pruritus, skin irritation, burning sensation, tingling, dry skin, erythema and rash, and calcium metabolism to a certain extent. Therefore, only a limited number of products, with significant effect and small side effects, are suitable for psoriasis patients.

Recent studies find that in normal human epidermal layers, the expression of epidermal growth factor receptor (EGFR) is inconsistent, and a large amount of EGFR is expressed in the actively dividing basal layers and part of the prickle cell layer near the basal layer. However, in the skin of someone with psoriasis vulgaris, EGFR is expressed in all the layers, particularly in the layers above the prickle cell layer. The expression level is eight times higher than the normal control. The overexpression of EGFR in the skin lesions of psoriatic vulgaris suggests that psoriasis may be associated with excessive proliferation of epidermal cells and abnormal differentiation. Controlling the abnormal expression of EGFR may open up new ways and provide new drugs for the treatment of psoriasis.

Tyrosine kinase receptors are transmembrane proteins involved in signal transduction, in which the receptors transduce the growth factor signals from the cell surface to intracellular molecules that control critical cellular functions, for example, cell growth, mutation, angiogenesis and apoptosis inhibition. Epidermal growth factor receptor (EGFR) tyrosine kinase is one type of such receptors. There has not been an effective tyrosine kinase inhibitor in the market for treating psoriasis nowadays.

### DESCRIPTION OF THE INVENTION

In light of the limitations of existing technology, the present invention provides topical pharmaceutical compositions inhibiting tyrosine kinase and the preparation methods thereof.

First, the present invention provides a topical skin pharmaceutical composition, which contains the active ingredient inhibiting tyrosine kinase and excipients of the topical preparation, wherein the active ingredient is Icotinib or a pharmaceutically acceptable salt thereof, and the excipients include dispersion mediums, emulsifiers and/or one or more other pharmaceutically acceptable excipients of the topical preparation.

The present invention also provides preferred technology for the above technical solutions:
Preferably, the active ingredient can be Icotinib freebase, Icotinib hydrochloride, Icotinib maleate or Icotinib phosphate;
Preferably, the concentration of the active ingredient is 0.1-11 wt%, more preferably 0.3-5 wt%, further preferably 0.9-4.3 wt%, and especially preferably 1-1.5 wt%;
Preferably, the dispersion mediums include water soluble matrices and/or oily matrices;
Preferably, the water soluble matrices include water, glycerin, gelatin, ethanol, polyethylene glycol, propylene glycol, DMSO and/or cellulose derivatives;
Preferably, the concentration of the water soluble matrices is 40-100 wt%, more preferably 65-85 wt%;
Preferably, the oily matrices include hydrocarbon matrices, oil matrices, lipid matrices and/or organosilicon oxide polymers;
Preferably, the hydrocarbon matrices may include hexadecanol, octadecanol and/or liquid paraffin; the oily matrices may include soybean oil, castor oil, glycerin mono-, di-stearate and/or Vaseline; the lipid matrices may include lanolin and/or beeswax; the organosilicon oxide polymers are dimethylsiloxane polymer;
Preferably, the percentage of the oily matrices is 0-25 wt%, or more preferably 9-11 wt%;
Preferably, the emulsifiers are anionic emulsifiers and/or nonionic emulsifiers, or more preferably nonionic emulsifiers;
Preferably, the anionic emulsifiers are monovalent soaps and/or fatty alcohol sulfates; the nonionic emulsifiers are higher fatty acid polyol esters, polyethylene glycol fatty acid esters and/or polyoxyethylene derivatives;
Preferably, the monovalent soap is sodium stearate; the fatty alcohol sulfates are sodium dodecyl sulfates and/or sodium dodecyl sulfates; the higher fatty acid polyol esters are hexadecanol, octadecanol, stearic acid monoglyceride, poloxamer, polysorbate-80, polysorbate-60 and/or polysorbate-85; the polyoxyethylene derivative is peregal O; the polyethylene glycol fatty acid esters are polyethylene glycol-7-stearate and/or polyethylene glycol glyceryl oleate;
Preferably, the concentration of the emulsifiers is 0-23 wt%, or more preferably 10-15 wt%;
Preferably, the other pharmaceutically acceptable excipients of the topical preparation are suspending agents;
Preferably, the suspending agents are polymeric suspending agents;
Preferably, the polymeric suspending agents are carbomer, polyvinylpyrrolidone (PVP-K30), glucan, sodium carboxymethylcellulose (CMC-Na), hydroxypropyl methyl cellulose (HPMC) and/or methyl cellulose;
Preferably, the percentage of the suspending agents is 0-8.5 wt%, or more preferably 0-0.1 wt%;
Preferably, the other pharmaceutically acceptable excipients of the topical preparation are pH regulators;
Preferably, the pH regulators are alkalis, acids and/or buffer solutions;
Preferably, the alkalis include sodium hydroxide, potassium hydroxide and/or ammonium hydroxide; the buffer solutions include the buffer pairs including, but not limited to weak alkalis and weak acids;
Preferably, the weak acids include citric acid, potassium acid phthalate and/or acetic acid; the weak alkalis include triethanolamine, diethanolamine, disodium hydrogen phosphate, sodium dihydrogen phosphate, sodium citrate and/or sodium acetate;
Preferably, the percentage of the PH regulators is 0-12.8 wt%, or more preferably 0.2-1.5 wt%;
Preferably, the other pharmaceutically acceptable excipients of the topical preparation are preservatives;
Preferably, the preservatives include p-hydroxybenzoic acid esters and/or sorbic acid and its salts;
Preferably, the preservatives include ethyl p-hydroxybenzoate, methyl p-hydroxybenzoate, propyl p-hydroxybenzoate, sorbic acid, potassium sorbate, chlorocresol and/or chlorobutanol;
Preferably, the percentage of the preservatives is 0-0.3 wt%;
Preferably, the other pharmaceutically acceptable excipients of the topical preparation are transdermal enhancers;
Preferably, the transdermal enhancers include Transcutol P and/or Labrasol; or
Preferably, the concentration of the transdermal enhancers is 0-45 wt%, or more preferably 15-30 wt%.

The invention also provides a use for the preparation of ointments from the topical pharmaceutical compositions.

Preferably, the ointment is cream.

The invention also provides a use for the preparation of gels from the topical pharmaceutical compositions.

Preferably, the gel is transparent gel.

The invention also provides a use for the preparation of medicines from the topical pharmaceutical compositions for the treatment of non-malignant excessive hyperplasia disorders or tumor and complications thereof.

The invention also provides preferred technology for the above the technical solutions:
Preferably, the non-malignant excessive hyperplasia disorder is benign skin hyperplasia;
Preferably, the non-malignant excessive hyperplasia disorder is dermatosis;
Preferably, the dermatosis may include psoriasis, scleroderma and/or dermatosis caused by diabetes;
Preferably, the dermatosis is psoriasis; or

Preferably, the tumor and complications thereof are dermatoma and its complications.

The invention also provides a method for treating mammalian tissue excessive hyperplasia disease, including administering a therapeutically effective amount of the pharmaceutical compositions to a patient suffering from tissue excessive hyperplasia disease.

The invention also provides preferred technologies for the above technical solutions:
Preferably, the tissue excessive hyperplasia disease can be dermatosis, dermatoma and/or its complications;
Preferably, the dermatosis can be psoriasis, scleroderma and/or dermatosis caused by diabetes; or
Preferably, the dermatosis is psoriasis.

In particular, the active ingredient of the skin topical pharmaceutical compositions provided by the invention can be Icotinib freebase, which is prepared by the following method: Icotinib hydrochloride was dissolved in a mixture of ethanol and water. The sodium hydroxide solution was added dropwise into the solution of Icotinib hydrochloride at a certain temperature until the pH value of the mixture is greater than or equal to 13. The reaction solution was stirred, cooled to room temperature, and precipitated. The precipitation was filtered, washed by pure water, and dried under vacuum below a certain temperature to obtain the Icotinib freebase. The Icotinib freebase of the present invention includes, but not limited to, the molecules prepared from the above preparation method. The structure of Icotinib freebase is as follows:

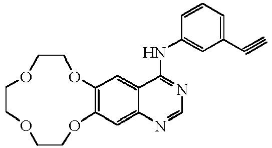

In particular, the active ingredient of the skin topical pharmaceutical compositions provided by the invention is Icotinib hydrochloride, preferably with the crystal form I of Icotinib hydrochloride, which includes, but is not limited to, the molecules prepared according to the preparation method disclosed in the international application of WO2010/003313A1. The structure of Icotinib hydrochloride is as follows:

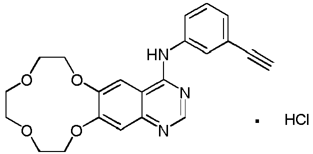

In particular, the active ingredient of the skin topical pharmaceutical compositions provided by the invention is Icotinib maleate, which can be prepared by the following method. First, Icotinib freebase was dissolved in acetone achieving the Icotinib solution. In addition, maleic acid was dissolved in acetone achieving the maleic acid solution. The maleic acid solution was added to the Icotinib solution, the reaction mixture stirred and reacted, and then Icotinib maleate was isolated. The Icotinib maleate includes, but is not limited to, the molecules prepared from the above preparation method. The structure of Icotinib maleate is as follows:

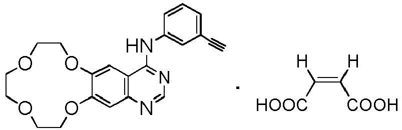

In particular, the active ingredient of the skin topical pharmaceutical compositions provided by the invention is Icotinib phosphate, which can be prepared by the following method. First, Icotinib freebase was dissolved in isopropanol, achieving the Icotinib solution. In addition, the phosphoric acid solution was added to isopropanol achieving the phosphoric acid solution. The phosphoric acid solution was added to the Icotinib solution, and the reaction mixture was stirred and isolated to obtain Icotinib phosphate. The Icotinib phosphate includes, but is not limited to, the molecules prepared from the above preparation method. The structure of Icotinib phosphate is as follows:

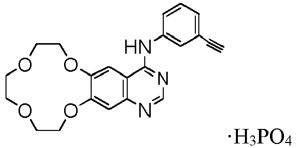

In particular, the Icotinib or its pharmaceutically acceptable salts thereof in the present invention refer to any pharmaceutically acceptable materials containing the structure of Icotinib, including, but not limited to: Icotinib freebase, Icotinib hydrochloride, Icotinib maleate, Icotinib phosphate, Icotinib solvate, Icotinib chelate, Icotinib hydrate, and all crystal forms of the above materials.

The Icotinib or pharmaceutically acceptable salts thereof in the invention can contain an asymmetric center, chiral axis and chiral surface (see pages 1119-1190 on "Stereochemistry of Carbon Compounds" (edited by E.X. Eliel and S.H. Wilen) published by John Wiley & Sons, Inc. in 1994), and can include racemate, racemic mixture, individual diastereomers, all possible isomers and their mixtures, including optical isomers and all stereo isomers included in the invention. In addition, tautomers can include the Icotinib or pharmaceutically acceptable salts thereof in the invention. And all the tautomers are also included in the scope of the present invention.

The Icotinib or pharmaceutically acceptable salts thereof in the invention can exist in a large number of different crystalline forms.

Any kind of drug for clinical use should be made in forms suitable for different medical and preventive applications. These forms are called dosage forms, while the dosage forms are known as pharmaceutical preparations. To develop and produce pharmaceutical dosage forms and preparations that are of high curative effect, of minimal side effect, easy to take, convenient for transportation and storage, and of stable quality, the production mainly depends on the pharmaceutical excipients, although it also relates to production technologies, production facilities, preparation processes, and quality controls and so on. For any kind of preparation, in addition to the active ingredients (main drug), the rest are pharmaceutical excipients. Therefore, the quality of pharmaceutical excipients, and the scientificity and rationality of the excipient formulas, directly affect the quality of the preparations.

The topical pharmaceutical compositions of the Icotinib or the pharmaceutically acceptable salts thereof should be made to allow the drug to be released through the epidermis to apply its therapeutic effect in the skin. The skin, by covering the whole body, prevents the body from losing water, electrolytes and other substances, and also is a barrier against the invasion of foreign substances, thereby playing an important role in protecting the body. The barrier function of the skin is mainly undertaken by the corneum, which is a thin film layer with a certain mechanical strength, and a major barrier for the transdermal absorption of the drug. It is generally believed that the corneum can hold a proper concentration of water-soluble and fat-soluble substances, and drugs with small molecular weight can diffuse into the endothecium through the intercellular space. Hair follicles and cryptae can penetrate the corneum, thereby providing another passway for the drug absorption.

There are many factors that affect the transdermal absorption of the drug, such as physiological factors, the physicochemical properties of drugs, and the type and composition of matrices. Although the inherent activity of the drug is the most important factor that determines its therapeutic use, the release and transdermal absorption of the drug are influenced by the matrices to a great extent.

Icotinib (with a chemical name of 4-[(3-ethynyl phenyl) amino] quinazoline [6, 7-b]-12-crown-4) or a pharmaceutically acceptable salt thereof is the active ingredient of the preparation.

Transcutol P has a chemical name of diethylene glycol monoethyl ether. It can be used as the solvent and transdermal absorption enhancer for the active drug in pharmaceutical manufacturing.

Labrasol, with a Chinese chemical name of caprylic capric acid macrogol glycerides and an English name of Caprylocaproyl macrogol-8 glycerides, is a mixture of mono-, di-, tri-glycerides and mono-, di-fatty acid polyethylene glycol ester with a certain ratio, which is commonly used as an emulsifier and a transdermal absorption enhancer in pharmaceutical manufacturing.

Carbopol, with a Chinese name of carbomer, is a white, "fluffy", acidic powder that can absorb water and has a slight special smell. It is a polymer of acrylic bonded allyl sucrose or pentaerythritol allyl ether, and is commonly used as a gel matrix.

Tefose 63^{®}, with a chemical name of polyethylene glycol-7 stearate, is a mixture of polyethylene glycol-6 stearate (PEG-6 stearate), ethylene glycol palmitostearate, and polyethylene glycol-32 stearate (PEG-32 stearate).

Labrafil M 1944^{®}, with a chemical name of oleic acid polyethylene glycol glyceride (EP), is a mixture composed of mono-, di-, tri-glycerides and mono-, di-fatty acid polyethylene glycol ester with a certain ratio.

DMSO, with a chemical name of dimethyl sulfoxide, is a colorless liquid. It can solve with many organic solvents and water.

To prepare the preparation provided by the invention, one needs to pay attention to the insolubility between the excipients, for example, carbomer being discolored when encountering m-dihydroxybenzene, and being insoluble with phenol, cationic polymers, strong acid, and electrolyte with high concentration. Trace amounts of iron or other transition metals are capable of catalyzing and degrading carbopol dispersions. Large amounts of heat can be produced when carbomer comes in contact with strong alkaline substances, such as ammonia, potassium hydroxide, sodium hydroxide, or strongly alkaline organic amine. Some amino-containing drugs can form soluble complexes with carbomer, and, generally speaking, the occurrence of such cases can be prevented by adjusting the solubility parameter of the liquid with appropriate alcohol or polyatomic acid.

The excipients and the insolubility between them in the preparations are common knowledge in the field, which can be found in "The Handbook of Pharmaceutical Excipients" (edited by [EN] R.C. Rowe, [US] P.J. Sheskey, and [EN] P.J. Weller, mainly translated by Junmin Zheng, and published by Chemistry Industry Press), "The Handbook of Commonly Used Pharmaceutical Excipients" (mainly edited by Jiewei Li, Jixiang Liu, and published by The Second Military Medical University Press) and "Pharmaceutical Excipients Daquan" (mainly edited by Mingsheng Luo, and Tianhui Gao, and published by Sichuan Science & Technology Press), none of which are explained in detail here.

The present invention provides topical preparations containing Icotinib or pharmaceutically acceptable salts thereof and the preparation methods. The topical preparations provided in the invention are with minimal skin irritation, or no adverse effects such as pruritus, burning sensations, tingling, dry skin, erythema, and rashes. The preparation will neither incur parahormone-related side effects such as skin atrophy, pigmentation or hypopigmentation after long-term use, nor cause any related dermatological symptoms after drug withdrawal. The preparation methods are easily understood, operable, and controllable, and suitable for industry mass production.

### MODES FOR CARRYING OUT THE INVENTION

The following examples shall be only used to illustrate specific embodiments of the present invention so that those skilled in the art can implement the present invention, but not used to limit the scope of the present invention. In the embodiments of the present invention, the techniques or methods, with no special instructions, are conventional techniques or methods in the field.

The embodiments of the topical preparations provided in the invention are as follows. The values in the embodiments mean "wt%" with no special instructions.

### Examples 1-10

### 1. Formulations (see Table 1)

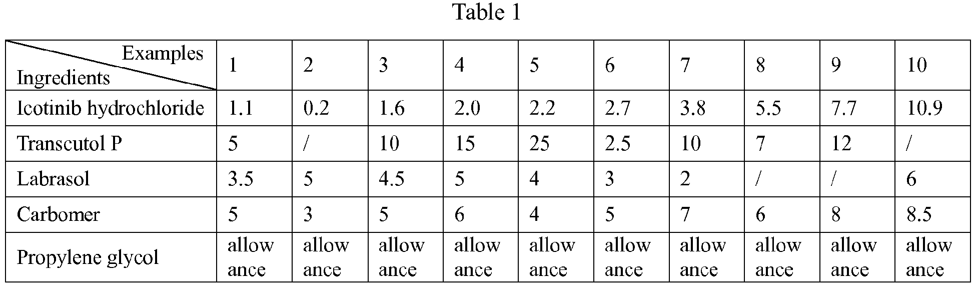

### 2. Preparation Methods of Examples 1-10

(1) The prescribed amount of carbomer was weighed, and fully swollen in propylene glycol;
(2) The prescribed amounts of Icotinib hydrochloride, Transcutol P and/or Labrasol were weighed individually and mixed evenly;
(3) The mixture obtained in step (2) was added to the swollen carbomer solution obtained in step (1);
(4) The mixture obtained in step (3) was stirred until it turned transparent at room temperature.

### Examples 11-14

### 1. Formulations (see Table 2)

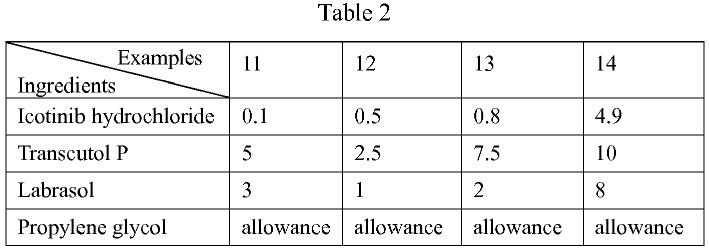

### 2. Preparation Methods of Examples 11-14

(1) The prescribed amounts of Icotinib hydrochloride, Transcutol P, and Labrasol were weighed individually and mixed evenly;
(2) The mixture obtained in step (1) was added to propylene glycol solution;
(3) The mixture obtained in step (2) was stirred until it turned transparent at room temperature.

### Examples 15-20

### 1. Formulations (see Table 3)

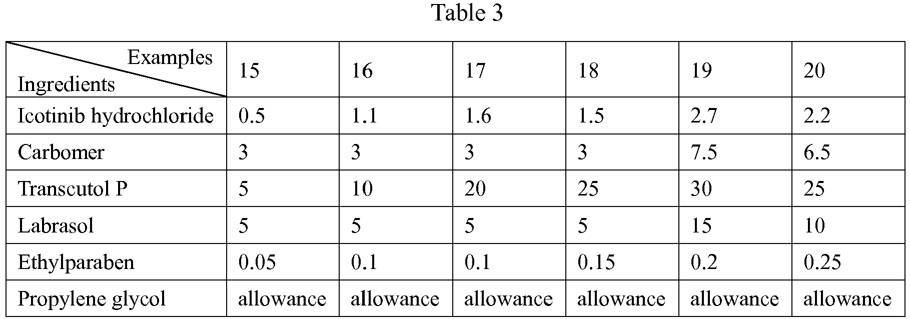

### 2. Preparation Methods of Examples 15-20

(1) The prescribed amounts of carbomer and ethylparaben were weighed individually, and fully swollen in propylene glycol;
(2) The prescribed amounts of Icotinib hydrochloride, Transcutol P, and Labrasol were weighed and mixed evenly;
(3) The mixture obtained in step (2) was added to the swollen carbomer solution obtained in step (1);
(4) The mixture obtained in step (3) was stirred to until it turned transparent at room temperature.

### Examples 21-24

### 1. Formulations (see Table 4)

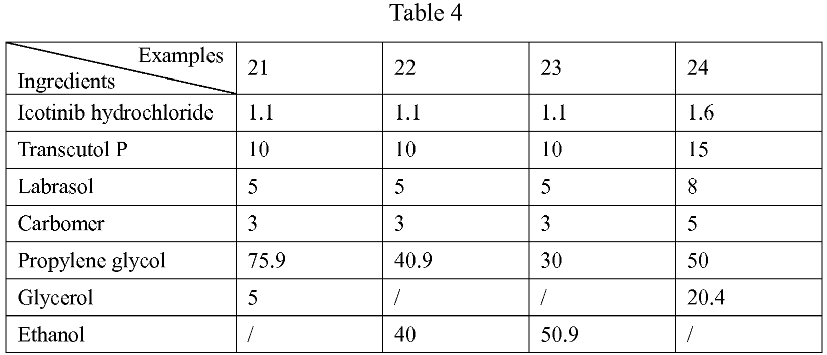

### 2. Preparation Methods of Examples 21-24

(1) The prescribed amount of carbomer was weighed, and fully swollen in propylene glycol;
(2) The prescribed amounts of Icotinib hydrochloride, Transcutol P, and Labrasol were weighed individually and mixed evenly;
(3) The mixture obtained in step (2) was added to the swollen carbomer solution obtained in step (1);
(4) The prescribed amount of glycerol or ethanol was added to the mixture obtained in step (3), and stirred until it turned transparent at room temperature.

### Examples 25-29

### 1. Formulations (see Table 5)

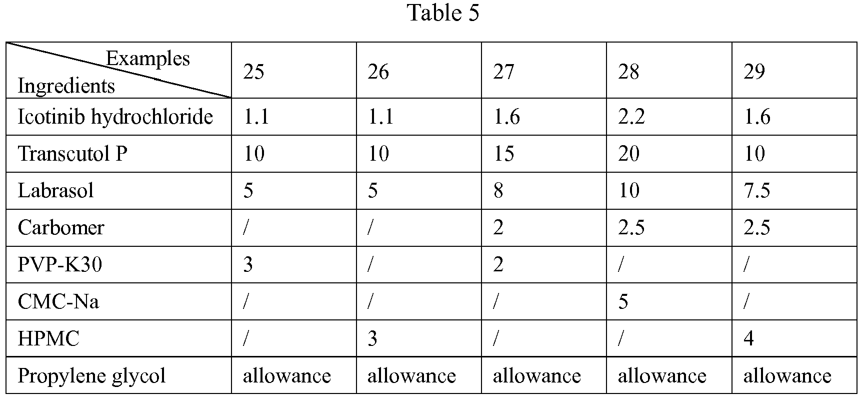

### 2. Preparation Methods of Examples 25-29

(1) The prescribed amounts of PVP-K30, carbomer, CMC-Na and/or HPMC were weighed individually, and fully swollen in propylene glycol;
(2) The prescribed amounts of Icotinib hydrochloride, Transcutol P, and Labrasol were weighed individually and mixed evenly;
(3) The mixture obtained in step (2) was added to the swollen carbomer solution obtained in step (1), and stirred until it turned transparent at room temperature.

### Examples 30-36

### 1. Formulations (see Table 6)

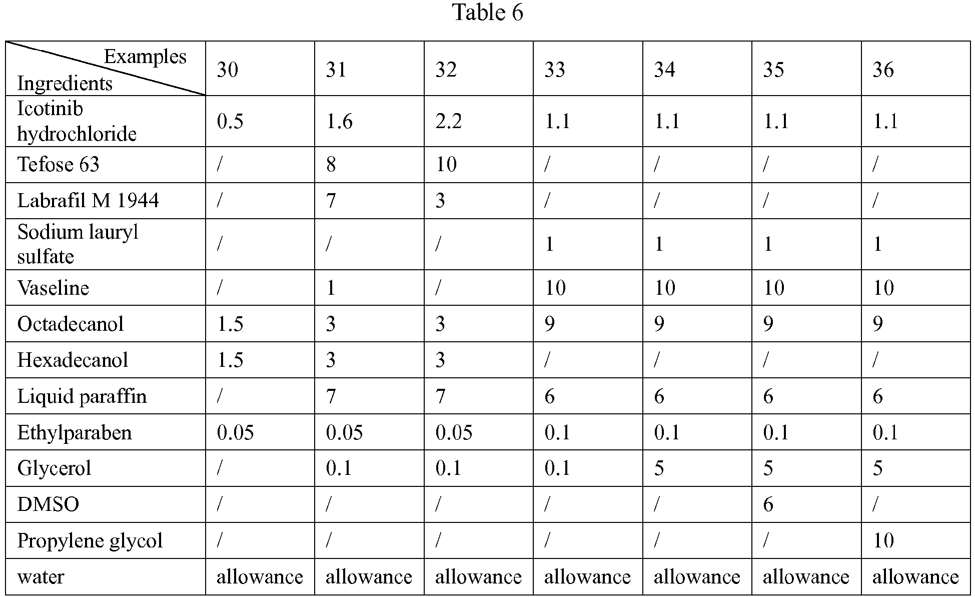

### 2. Preparation Methods

### A, Preparation Methods of Examples 30-32

(1) The prescribed amounts of Tefose 63, Labrafil M 1944, Vaseline, octadecanol, hexadecanol, ethylparaben and/or liquid paraffin were weighed individually, mixed, stirred, and heated to 75-80°C, until diatexis;
(2) In addition, the prescribed amounts of Icotinib hydrochloride, water and/or glycerol were weighed individually, mixed, heated to 60-70°C, and stirred evenly;
(3) The mixture obtained in step (2) was added to the mixture obtained in step (1), emulsified for 20 minutes, and cooled to room temperature in a water bath.

### B, Preparation Methods of Examples 33-36

(1) The prescribed amounts of Vaseline, octadecanol, liquid paraffin and ethylparaben were weighed individually, mixed, stirred, and heated to 75-80°C, until diatexis;
(2) In addition, the prescribed amounts of glycerol, sodium lauryl sulfate, water, DMSO and/or propylene glycol, mixed, heated to 60-70°C, and stirred evenly;
(3) The mixture obtained in step (2) was added to the mixture obtained in step (1), and emulsified for 10-30 minutes;
(4) The prescribed amount of Icotinib hydrochloride was added to the mixture obtained in step (3), emulsified sequentially for 10 minutes, and cooled to room temperature.

### Examples 37-43

### 1. Formulations (see Table 7)

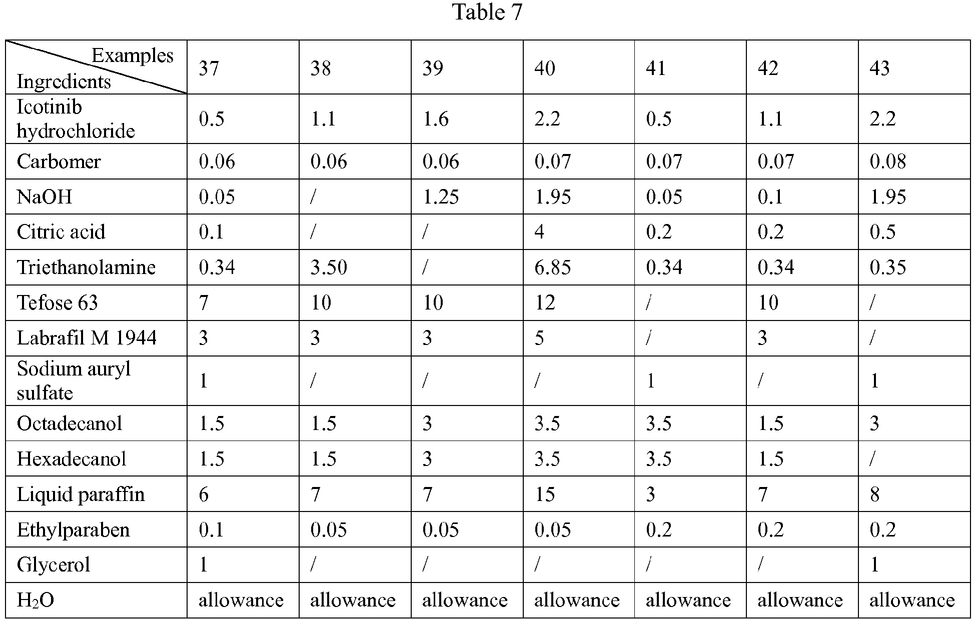

### 2. Preparation Methods of Examples 37-43

(1) The prescribed amount of carbomer was weighed, dissolved in water, added to triethanolamine, and stirred until fully swollen;
(2) In addition, the prescribed amount of Icotinib hydrochloride was weighed, added with water, stirred at 40°C, added with the prescribed amount of sodium hydroxide, and stirred evenly;
(3) In addition, the prescribed amounts of citric acid and/or the rest of the triethanolamine were weighed, dissolved in water, added to the solution obtained in step (2) dropwise, added with the carbomer solution obtained in step (1), emulsified for 5 minutes, and then heated to 60-70°C ;
(4) In addition, the prescribed amounts of Tefose 63, Labrafil M 1944, sodium lauryl sulfate, octadecanol, hexadecanol, liquid paraffin, ethylparaben and/or glycerol were weighed individually, mixed, stirred, heated to 75-80°Cuntil diatexis, added to the solution obtained in step (3), emulsified for 15 minutes, stirred in a water bath, and cooled to room temperature.

### Examples 44-50

### 1. Formulations (see Table 8)

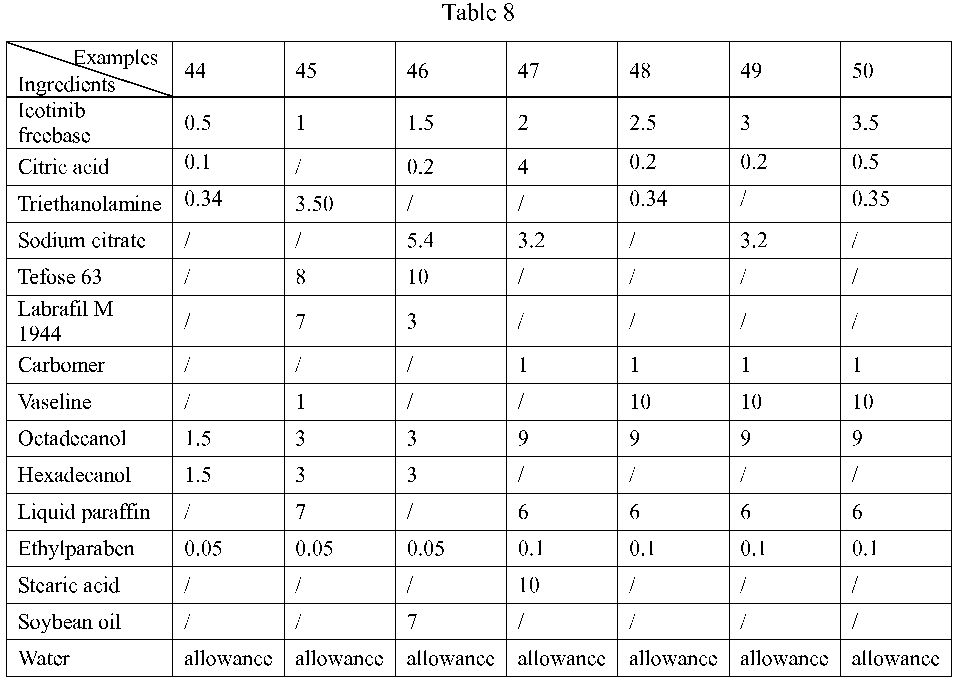

### 2. A, Preparation Methods of Examples 44-47

(1) The prescribed amounts of Tefose 63, Labrafil M 1944, Vaseline, octadecanol, hexadecanol, soybean oil, stearic acid, ethylparaben and/or liquid paraffin were weighed individually, mixed, stirred, and heated to 75-80°C until diatexis;
(2) In addition, the prescribed amounts of Icotinib freebase, triethanolamine, citric acid, sodium citrate, water and/or carbomer were weigh individually, mixed, stirred, and heated to 60-70°C ;
(3) The mixture obtained in step (2) was added to the mixture obtained in step (1), emulsified for 20 minutes, and cooled to room temperature in a water bath.

### B, Preparation Methods of Examples 48-50

(1) The prescribed amounts of Vaseline, octadecanol, liquid paraffin and ethylparaben were weighed individually, mixed, stirred, and heated to 75-80°C until diatexis;
(2) In addition, the prescribed amounts of citric acid, carbomer, water and/or triethanolamine were weighed individually, mixed, stirred, and heated to 60-70°C;
(3) The mixture obtained in step (2) was added to the mixture obtained in step (1), and emulsified for 10 minutes;
(4) The prescribed amount of Icotinib freebase was added to the mixture obtained in step (3), sequentially emulsified for 10 minutes, and cooled to room temperature.

### Examples 51-56

### 1. Formulations (see Table 9)

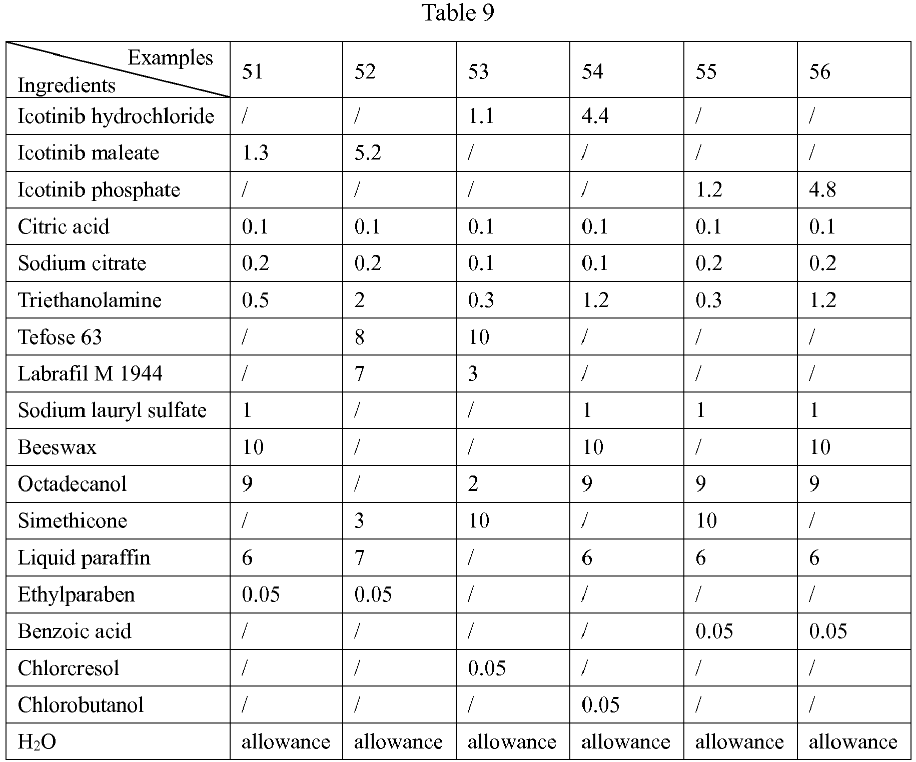

### 2. Preparation Methods of Examples 51-56

(1) The prescribed amounts of Tefose 63, Labrafil M 1944, beeswax, simethicone, octadecanol, liquid paraffin, ethylparaben, benzoic acid, chlorobutanol and/or chlorcresol were weighed individually, mixed, stirred, and heated to 75-80°C until diatexis;
(2) In addition, the prescribed amounts of Icotinib hydrochloride, Icotinib maleate, Icotinib phosphate, triethanolamine, citric acid, sodium citrate, water and/or sodium lauryl sulfate were weighed individually, mixed, stirred, and heated to 60-70°C;
(3) The mixture obtained in step (2) was added to the mixture obtained in step (1), emulsified for 20 minutes, and cooled to room temperature.

### Example 57. Preparation of Icotinib freebase

100g Icotinib hydrochloride was dissolved in the mixture of 300ml ethanol and 200ml water. A solution of 11.2g sodium hydroxide in 100ml water was added dropwise at 60°C to the Icotinib hydrochloride solution until the pH value of the reaction solution reached 13. The reaction solution was then stirred for an hour and then cooled to room temperature. The precipitate was filtered and washed with purified water and dried for 8 hours under vacuum below 60°C to obtain 90g Icotinib freebase.

### Example 58. Preparation of Icotinib Maleate

10mg Icotinib freebase was dissolved in 1ml acetone to obtain Icotinib solution. In addition, 34.82mg maleic acid was dissolved in 3ml acetone to obtain a 0.1mol/L maleic acid solution. 0.26ml of the 0.1mol/L maleic acid solution was added to the Icotinib solution and the reaction mixture was stirred for 24 hours to obtain Icotinib maleate.

### Example 59. Preparation of Icotinib Phosphate

10mg Icotinib freebase was dissolved in 1ml isopropanol to obtain Icotinib solution. In addition, 18.9µL phosphoric acid was dissolved in 3ml isopropanol to obtain a 0.1mol/L phosphoric acid solution. 0.26ml of the 0.1mol/L phosphoric acid solution was added to the Icotinib solution and the reaction mixture was stirred for 24 hours, and then the Icotinib phosphate was isolated.

### Example A. The experiment on the effects of Icotinib hydrochloride cream on mouse tail epidermis granular layer formation

### Test Materials:

Test drugs: Icotinib hydrochloride cream preparations, with the specification of 1g/100ml (1%);
Negative control cream matrix group: the group does not contain active ingredients (Icotinib hydrochloride), but contains the other ingredients are the same as the test drug;
Positive control drugs: Halometasone Cream, with the specification of 15g: 7.5mg, Hongkong Aomei pharmaceutical factory, with the batch number of: 0911501;
Test animals: ICR mice, male, 30;
Test instrument: OLYMPUS biological microscope.

### Test Method:

30 ICR male mice were randomly divided into 3 groups, with 10 mice in each group: the negative control cream matrix group, the positive control drug group of Halometasone Cream, and the 1% Icotinib hydrochloride cream group. The creams were applied to the tail skin of the mice. Before each administration, the mice tails were gently wiped with a cotton swab and water, and the mice tails in different groups were applied with a thin layer of different drugs, 2 times a day, for 14 consecutive days. After treatment the mice were killed, and the skin with a size of 1.5cm×0.2cm was taken at 1-2cm from the distal end of the mouse tail, fixed with 4% formalin, embedded with paraffin, sliced, and stained with Hematoxylin and Eosin ("H&E").

The changes of skin keratosis layer, the granular layer, the prickle cell layer, the basal cell layer, the dermis, the follicles and other changes of the mouse tail were observed under a light microscope. The continuous granular layer cells that exist between any two adjacent follicular epidermises (i.e., scale epidermis) are called scales with granular layer. 50 scales were observed for each animal, and the scales with the formation of granular layer (SG) were calculated. The data are shown in table A.

**Table A: The effects of different drugs on the differentiation of mouse tail epidermis (the formation of granular layer)**

| Groups | *N* | SG/50 |
|---|---|---|
| Negative control cream matrix group | 10 | 2.2±1.4 |
| Halometasone Cream group | 10 | 3.6±2.0 |
| 1% Icotinib hydrochloride cream group | 10 | 7.8±4.1** |

| | | |
|---|---|---|
| Note: compared with the negative control matrix group, **P<0.01 | | |

As shown from table A: Icotinib hydrochloride cream preparation could significantly promote the formation of scale granular layers on the mouse tail compared with the negative control matrix group, and the effect is stronger than the positive control group of Halometasone Cream.

### Example B. The experiment on the effects of Icotinib hydrochloride gel on the formation of mouse tail epidermis granular layer

### Test Materials:

Test drugs: Icotinib hydrochloride gel preparation: with the specification of 1g/100ml (1%); and the specification of 1.5g/100ml (1.5%);
Negative control gel matrix group: the group does not contain active ingredients (Icotinib hydrochloride), but the other ingredients are the same as the test drug;
Positive control drugs: Clobetasonl Propionate Cream, with the specification of 10g: 2mg (0.02%), Guangdong Shunfeng Pharmaceutical Co. Ltd., with the batch number of: 20110304;
Test animals: ICR mice, male, 40;
Test instrument: biological microscope.

### Test Method:

40 ICR male mice were randomly divided into 4 groups, with 10 mice in each group: the negative control gel matrix group, the 1% Icotinib hydrochloride gel group, the 1.5% Icotinib hydrochloride gel group, and the positive drug Clobetasonl Propionate Cream group. The creams were applied to the tail skin of the mice. Before each administration, the mice tails were gently wiped with a cotton swab and water, and the mice tails in different groups were applied with a thin layer of different drugs, 2 times a day, for 14 consecutive days. After treatment the mice were killed, and the skin with a size of 1.5cm×0.2cm was taken at 1-2cm from the distal end of the mice tails, fixed with 4% neutral formalin, embedded with paraffin, sliced, and stained with H&E.

The changes of the skin keratosis layer, the granular layer, the prickle cell layer, the basal cell layer, the dermis, the follicles and other changes of the mouse tail were observed under a light microscope. The continuous granular layer cells exist between any two adjacent follicular epidermises (i.e., scale epidermis) are called scales with granular layer. Each animal was observed, and the ratios of the number of the scales with the formation of granular layer to the number of the total scales were calculated. The data are shown in table B.

**Table B: The effects of different drugs on the differentiation of mouse tail epidermis (the formation of granular layer)**

| Groups | *N* | Ratio% |
|---|---|---|
| Negative control gel matrix group | 10 | 4.8±3.2 |
| 1% Icotinib hydrochloride gel group | 10 | 11.1±3.9** |
| 1.5% Icotinib hydrochloride gel group | 10 | 13.2±4.2** |
| Clobetasonl Propionate Cream | 10 | 10.1±4.0** |

| | | |
|---|---|---|
| Note: compared with the negative control matrix group, **P<0.01 | | |

As shown from table B: the 1% Icotinib hydrochloride gel group and 1.5% Icotinib hydrochloride gel group all have the effect of increasing the number of the scales with granular layer on mice tails.

### Example C. The experiment on the effects of Icotinib hydrochloride cream on the ear skin psoriasis-like lesions model of cavy induced by propranolol hydrochloride

### Test Materials:

Test drugs: Icotinib hydrochloride cream preparation: with the specification of 1g/100ml (1%); with the specification of 2g/100ml (2%); and with the specification of 4g/100ml (4%);
Negative control cream matrix group: the group does not contain active ingredients (Icotinib hydrochloride), but the other ingredients are the same as the test drug;
Positive control drugs: Calcipotriol Ointment, with the batch number of EH4129, LEO Laboratories Ltd.;
Test animals: cavies, 250-300g, male and female each half, 70;
Test instrument: biological microscope.

### Test Method:

5% propranolol liniment: 5g propranolol hydrochloride was dissolved in an appropriate amount of 50% ethanol, added with 5ml laurocapram (Azone-propylene glycol) as composite accelerator, added with 5g polyvinylpyrrolidone (PVP-K30) as film-forming material, added with 50% ethanol to reach 100ml in the final mixture, and mixed.

For the 70 healthy adult cavies, 10 cavies were randomly selected (male and female each half) as normal control group. For the rest of the cavies, the dorsal skins on both sides of auriculars were applied with 5% propranolol hydrochloride emulsion liniment (80µl/ear), once every morning and once every evening, for 2 consecutive weeks. After the completion of the modeling, the cavies were randomly divided into 6 groups, namely the model group, the negative control matrix group, the 1% Icotinib hydrochloride cream group, the 2% Icotinib hydrochloride cream group, the 4% Icotinib hydrochloride cream group, and the Calcipotriol Ointment group. Left and right ears of the animals in the model group were not applied with drugs. The left and right ears of the animals in the negative control matrix group were applied with the negative control matrix. The left and right ears of the animals in the administration group were applied with the drugs. After treatment for 2 weeks, the animals were killed with CO₂ anesthesia, and the skin with a size of 10mm×5mm was taken at the middle from both sides of auriculas of the animals in each group, fixed with 4% neutral formalin, embedded with paraffin, and stained with H&E. The changes of the skin keratosis layer, the granular layer, the prickle cell layer, the basal cell layer, the dermis, and other changes of the cavies' auriculas were observed under a light microscope. The thickness of the epidermal layer was measured under the light microscope at 100 x magnification. The grid micrometer with 25×25 grids number was counted. The thickness (mm) =grids number×0.0212. .

The changes of skin keratosis layer, the granular layer, the prickle cell layer, the basal cell layer, the dermis, and other changes of the cavies auriculas were observed under light microscope, and the thickness of the epidermal layer was measured with 100 times light microscope, the grids number of 25×25 grid micrometer being counted, grids number×0.0212=thickness (mm), the data being shown in Table C. (Note: an animal died in each group of the normal control group and 2% Icotinib hydrochloride cream group, and a slice of an animal from each of the model group and the calcipotriol group was not processed properly for observation.)

**Table C: The effects of Icotinib hydrochloride cream on the thickness of cavies auriculas epidermis**

| Groups | Groups | *N* | The thickness of epidermis/mm |
|---|---|---|---|
| 1 | Normal control group | 9 | 0.0538±0.0050^{ΔΔ} |
| 2 | Model group | 9 | 0.1457±0.0076** |
| 3 | Negative control matrix group | 10 | 0.1505±0.0179** |
| 4 | 1% Icotinib hydrochloride cream group | 10 | 0.0969±0.0130**^{ΔΔ} |
| 5 | 2% Icotinib hydrochloride cream group | 9 | 0.0904±0.0151**^{ΔΔ} |
| 6 | 4% Icotinib hydrochloride cream group | 10 | 0.0802±0.0199**^{ΔΔ} |
| 7 | Calcipotriol Ointment group | 9 | 0.1266±0.0256**^{ΔΔ} |

| | | | |
|---|---|---|---|
| Note: ** *P*<0.01, vs. Normal control group, ^{ΔΔ}*P*<0.01, vs. negative control matrix group, all data (Mean±SD) were carried on one-way analysis of variance with SPSS 17.0. | | | |

*P* value:
Group 2 vs. Group 3=0.560; Group 2 vs. Group 4=0.000; Group 2 vs. Group 5=0.000; Group 2 vs. Group 6=0.000; and Group 2 vs. Group 7=0.008;
Group 3 vs. Group 4=0.000; Group 3 vs. Group 5=0.000; Group 3 vs. Group 6=0.000; and Group 3 vs. Group 7=0.001;
Group 4 vs. Group 5=0.250; Group 4 vs. Group 6=0.003; and Group 4 vs. Group 7=0.000;
Group 5 vs. Group 6=0.075; Group 5 vs. Group 7=0.000.

One week after modeling, the cavies lost hair on the ear, and the local skin was red and swollen, and covered with a small amount of fine silver white scales. During the first and second weeks after treatment, on the cavies' auriculas appeared erosion, crusting, and peeling, while both ears of the animals of the control group were normal. After treatment for 2 weeks, on both ears of the animals appeared skin erosion and peeling in the model group, while the conditions for animals in the treatment group were improved. Particularly, the new hair was observed on the ear surface for the animals in the Icotinib hydrochloride cream treatment group, while no new hair was observed in the model group.

Under the light microscope, in the normal control group, the epidermal horny layers of the animals were completely homogeneous, the granular layer appeared single linear, the basal layer was composed of single columnar cells, the dermo-epidermis junction appeared wavy, the capillary vessels were in absence of congestion, and the structure appeared normal. In comparison, in the model group, the horny layer of the animals appeared akeratosis or hyperkeratosis, the prickle cell layer thickened, the trochanterellus extended, appearing club-shaped, the dermal papillae extended upwards, appearing rod-shaped, and the thickness of the ear epidermis significantly increased compared with the normal control group (P<0.01).

After administration of 1%, 2%, and 4% Icotinib hydrochloride cream, hyperkeratosis or akeratosis was lessened, the prickle cell layer appeared thinner, the trochanterellus extension and the dermal papillae upward extension were significantly reduced, and the thickness of the epidermis was significantly decreased compared with the model group (P<0.01).

Comparing the 2% cream group with the 1% cream group, there was no significant difference in the reduction of the epidermis thickness (P=0.250). Comparing the 4% cream group with the 1% cream group, the thickness of epidermis was significantly reduced (P<0.01).

The above-described examples are only for a full illustration of the embodiments of the present invention. The scope of the present invention is defined by the appended claims, but not limited to the embodiments. It is to be noted that various changes and modifications may be well known to those skilled in the art, and such changes and modifications are understood to be included within the scope of the present invention.

## Claims

1. A topical skin pharmaceutical composition, comprising an active ingredient capable of inhibiting tyrosine kinase and an excipient suitable for topical preparation,
wherein the active ingredient comprises Icotinib or a pharmaceutically acceptable salt thereof;
wherein the excipient comprises a dispersion medium, an emulsifier or one or more pharmaceutically acceptable excipient of the topical preparation.

2. The pharmaceutical composition according to claim 1, wherein the active ingredient comprises Icotinib freebase, Icotinib hydrochloride, Icotinib maleate or Icotinib phosphate.

3. The pharmaceutical composition according to claim 1 or 2, wherein the concentration of the active ingredient is 0.1-11 wt%.

4. The pharmaceutical composition according to anyone of claims 1-3, wherein the concentration of the active ingredient is 0.3-5 wt%.

5. The pharmaceutical composition according to anyone of claims 1-4, wherein the concentration of the active ingredient is 0.9-4.3 wt%.

6. The pharmaceutical composition according to anyone of claims 1-5, wherein the dispersion medium comprises a water soluble matrix, or an oily matrix.

7. The pharmaceutical composition according to claim 6, wherein the water soluble matrix is selected from a group consisting of water, glycerin, gelatin, ethanol, polyethylene glycol, propylene glycol, DMSO, and a cellulose derivative.

8. The pharmaceutical composition according to claim 6 or 7, wherein the concentration of the water soluble matrix is 40-100 wt%.

9. The pharmaceutical composition according to anyone of claims 6-8, wherein the concentration of the water soluble matrix is 65-85 wt%.

10. The pharmaceutical composition according to claim 6, wherein the oily matrix is selected from a group consisting of a hydrocarbon matrix, an oil matrix, a lipid matrix and a silicon oxide polymer.

11. The pharmaceutical composition according to claim 10,
wherein the hydrocarbon matrix is selected from a group consisting of hexadecanol, octadecanol and liquid paraffin;
wherein the oil matrix is selected from a group consisting of soybean oil, castor oil, glycerin mono-, di-stearate and Vaseline;
wherein the lipid matrix comprises lanolin or beeswax;
wherein the silicon oxide polymer is a dimethylsiloxane polymer.

12. The pharmaceutical composition according to claim 10 or 11, wherein the concentration of the oily matrix is 0-25 wt%.

13. The pharmaceutical composition according to anyone of claims 10-12, wherein the concentration of the oily matrix is 9-11 wt%.

14. The pharmaceutical composition according to anyone of claims 1-13, wherein the emulsifier comprises an anionic emulsifier or a nonionic emulsifier.

15. The pharmaceutical composition according to claim 14, wherein the anionic emulsifier comprises a monovalent soap or a fatty alcohol sulfate;
wherein the nonionic emulsifier comprises a higher fatty acid polyol ester, a polyethylene glycol fatty acid ester or a polyoxyethylene ether derivative.

16. The pharmaceutical composition according to claim 15, wherein the monovalent soap is sodium stearate;
wherein the fatty alcohol sulfate comprises sodium dodecyl sulfate or sodium cetyl sulfate;
wherein the higher fatty acid polyol ester is selected from a group consisting of hexadecanol, octadecanol, stearic acid monoglyceride, poloxamer, polysorbate-80, polysorbate-60, and polysorbate-85;
wherein the polyoxyethylene ether derivative is peregal O;
wherein the polyethylene glycol fatty acid ester comprises polyethylene glycol-7-stearate or polyethylene glycol glyceryl oleate.

17. The pharmaceutical composition according to anyone of claims 1-16, wherein the concentration of the emulsifier is 0-23 wt%.

18. The pharmaceutical composition according to anyone of claims 1-17, wherein the concentration of the emulsifier is 10-15 wt%.

19. The pharmaceutical composition according to anyone of claims 1-18, wherein the pharmaceutically acceptable excipient of the topical preparation is a suspending agent.

20. The pharmaceutical composition according to claim 19, wherein the suspending agent is a polymer suspending agent.

21. The pharmaceutical composition according to claim 20, wherein the polymer suspending agent is selected from a group consisting of carbomer, polyvinylpyrrolidone, glucan, sodium carboxymethylcellulose, hydroxypropyl methyl cellulose, and methyl cellulose.

22. The pharmaceutical composition according to anyone of claims 19-21, wherein the concentration of the suspending agent is 0-8.5 wt%.

23. The pharmaceutical composition according to anyone of claims 19-22, wherein the concentration of the suspending agent is 0-0.1 wt%.

24. The pharmaceutical composition according to anyone of claims 1-23, wherein the pharmaceutically acceptable excipient of the topical preparation is a pH regulator.

25. The pharmaceutical composition according to claim 24, wherein the pH regulator comprises an alkali, an acid or a buffer solution.

26. The pharmaceutical composition according to claim 25, wherein the alkali is selected from the group consisting of sodium hydroxide, potassium hydroxide and ammonium hydroxide;
wherein the buffer solution comprises a buffer pair consisting of a weak alkali and a weak acid.

27. The pharmaceutical composition according to claim 26, wherein the weak acid is selected from the group consisting of citric acid, potassium acid phthalate and acetic acid;
wherein the weak alkali is selected from the group consisting of triethanolamine, diethanolamine, disodium hydrogen phosphate, sodium dihydrogen phosphate, sodium citrate and sodium acetate.

28. The pharmaceutical composition according to anyone of claims 24-27, wherein the concentration of the PH regulator is 0-12.8 wt%.

29. The pharmaceutical composition according to anyone of claims 24-28, wherein the concentration of the PH regulator is 0.2-1.5 wt%.

30. The pharmaceutical composition according to anyone of claims 1-29, wherein the pharmaceutically acceptable excipient of the topical preparation is a preservative.

31. The pharmaceutical composition according to claim 30, wherein the preservative is selected from the group consisting of p-hydroxybenzoic acid esters and sorbic acid and its salt.

32. The pharmaceutical composition according to claim 30 or 31, wherein the preservative is selected from the group consisting of ethyl p-hydroxybenzoate, methyl p-hydroxybenzoate, propyl p-hydroxybenzoate, sorbic acid, potassium sorbate, chlorocresol and chlorobutanol.

33. The pharmaceutical composition according to anyone of claims 30-32, wherein the concentration of the preservative is 0-0.3 wt%.

34. The pharmaceutical composition according to anyone of claims 1-33, wherein the pharmaceutically acceptable excipient of the topical preparation is a transdermal enhancer.

35. The pharmaceutical composition according to claim 34, wherein the transdermal enhancer is selected from the group consisting of Transcutol P and Labrasol.

36. The pharmaceutical composition according to claim 34 or 35, wherein the concentration of the transdermal enhancer is 0-45 wt%.

37. The pharmaceutical composition according to anyone of claims 34-36, wherin the concentration of the transdermal enhancer is 15-30 wt%.

38. The pharmaceutical composition according to anyone of claims 1-37, wherein the pharmaceutical composition is an ointment.

39. The pharmaceutical composition according to claim 38, wherein the ointment is cream.

40. The pharmaceutical composition according to anyone of claims 1-37, wherein the pharmaceutical composition is a gel.

41. The pharmaceutical composition according to claim 40, wherein the gel is a transparent gel.

42. Use of the pharmaceutical composition of anyone of claims 1-41 for the preparation of medicines for the treatment of a non-malignant excessive hyperplasia disorder or tumor and complications thereof.

43. The use according to claim 42, wherein the non-malignant excessive hyperplasia disorder is skin benign hyperplasia.

44. The use according to claim 42 or 43, wherein the non-malignant excessive hyperplasia disorder is dermatosis.

45. The use according to claim 44, wherein the dermatosis comprises psoriasis, scleroderma, or dermatosis caused by diabetes.

46. The use according to claim 44 or 45, wherein the dermatosis is psoriasis.

47. The use according to claim 42, wherein the tumor and complications thereof are dermatoma and its complications.

48. A method for treating a mammalian tissue excessive hyperplasia disease, comprising administering a therapeutically effective amount of the pharmaceutical composition of anyone of claims 1-41 to a patient suffering from the tissue excessive hyperplasia disease.

49. The method for treating the mammalian tissue excessive hyperplasia disease according to claim 48, wherein the tissue excessive hyperplasia disease comprises dermatosis or dermatoma and its complications.

50. The method for treating the mammalian tissue excessive hyperplasia disease according to claim 49, wherein the dermatosis comprises psoriasis., scleroderma or dermatosis caused by diabetes.

51. The method for treating the mammalian tissue excessive hyperplasia disease according to claim 49 or 50, wherein the dermatosis is psoriasis.
